Europäisches Patentamt

**European Patent Office**

**Office européen des brevets**

(11) Publication number: **0 190 107**
**A2**

# (12) EUROPEAN PATENT APPLICATION

(21) Application number: **86830020.3**

(22) Date of filing: **24.01.86**

(51) Int. Cl.⁴: **A 61 N 5/06**

(30) Priority: **30.01.85 IT 4000985**

(43) Date of publication of application:
**06.08.86 Bulletin 86/32**

(84) Designated Contracting States:
**DE FR GB**

(71) Applicant: **Salveti, Claudio**
**Via Dante N. 3**
**I-43100 Parma(IT)**

(72) Inventor: **Salveti, Claudio**
**Via Dante N. 3**
**I-43100 Parma(IT)**

(74) Representative: **Bonfreschi, Mario**
**Bugnion S.p.A. Viale Trento Trieste, 25**
**I-41100 Modena(IT)**

(54) **Laser equipment for medical use.**

(57) In laser equipment as disclosed, which is intended for medical use, a number of laser devices (1) of the semiconductor type are ranged in succession one alongside the next such that the axes of the relative beams (2) emitted are disposed substantially parallel and lie within a common plane; the beams are directed at a focusing medium of elongated form embodied as a cylindrical lens (3) the longitudinal axis of which lies perpendicular to the parallel axes of the laser beams (2). The lens (3) focuses the beams (2) emitted by the laser devices (1) into a corresponding number of emergent beams (5) the cross-sections (6) of which each exhibit one dimension significantly greater than the other. The beam cross-sections lie in close proximity one to the next, aligned end-to-end through their longitudinal dimension, thereby forming what is substantially a continuous section of laser radiation in the form of a thin strip (7).

Fig.3

## Laser equipment for medical use

The invention relates to laser equipment for use in medical treatment, and is intended specifically, though by no means exclusively, for therapy whereby localized biostimulation of body tissue is brought about.

The prior art embraces laser equipment for medical use, in which the laser beam is generated from a gas source such as He-Ne, or by semiconductors (solid state).

In many laser therapy applications, the need exists for exposure of a given surface area, great or small in extent, to the influence of the beam; the medical laser equipment designed to meet such surface treatment requirements (notably that utilizing He-Ne) is provided with devices that move the laser beam about in such a way as to "scan" the treated area. Generally considered, such lasers present the joint disadvantage of being costly and, in many instances, impractical to use.

Essentially, the invention as disclosed and claimed herein sets out to eliminate the above drawbacks by providing laser equipment for medical use which is also capable of supplying greater output per unit of surface area than given hitherto by laser equipment adopted for similar applications (equipment utilizing a gas source, for example).

A preferred embodiment of the invention will.now be described in detail, by way of example, with the aid of the accompanying drawings, in which:

-fig 1 is a schematic representation of the invention, viewed frontally and in vertical elevation;

-fig 2 is s schematic representation of the section through I-I in fig 1;

-fig 3 is a schematic representation of fig 1, seen in perspective.

In equipment as illustrated in the above drawings, a number of laser devices 1 of the semiconductor type are ranged in succession one alongside the next in such a way that the axes of the beams 2 emitted are disposed parallel, lying within a common plane.

The laser beams 2 are directed at a focusing medium of elongated form consisting of a cylindrical lens 3 which, in the example illustrated, is of circular cross-section, and is disposed such that its longitudinal axis coincides with the plane containing the laser beams 2, and lies perpendicular to the beams themselves.

The purpose of such a cylindrical lens 3 is to focus incident laser beams 2 into a corresponding number of emergent beams 5 in such a way that the typical cross-section 6 of each emergent beam exhibits one dimension (length) significantly greater than the other (width).

The laser devices 1 and the cylindrical lens 3 are positioned in relation to one another in such a way that the emergent beams 5 occupy a common plane, and

0190107

the relative cross-sections 6 lie in close proximity one to the next; more precisely, the cross-sections will be aligned in an end-to-end succession through their longitudinal dimension, thereby forming what is substantially a continuous section comparable to a thin strip 7.

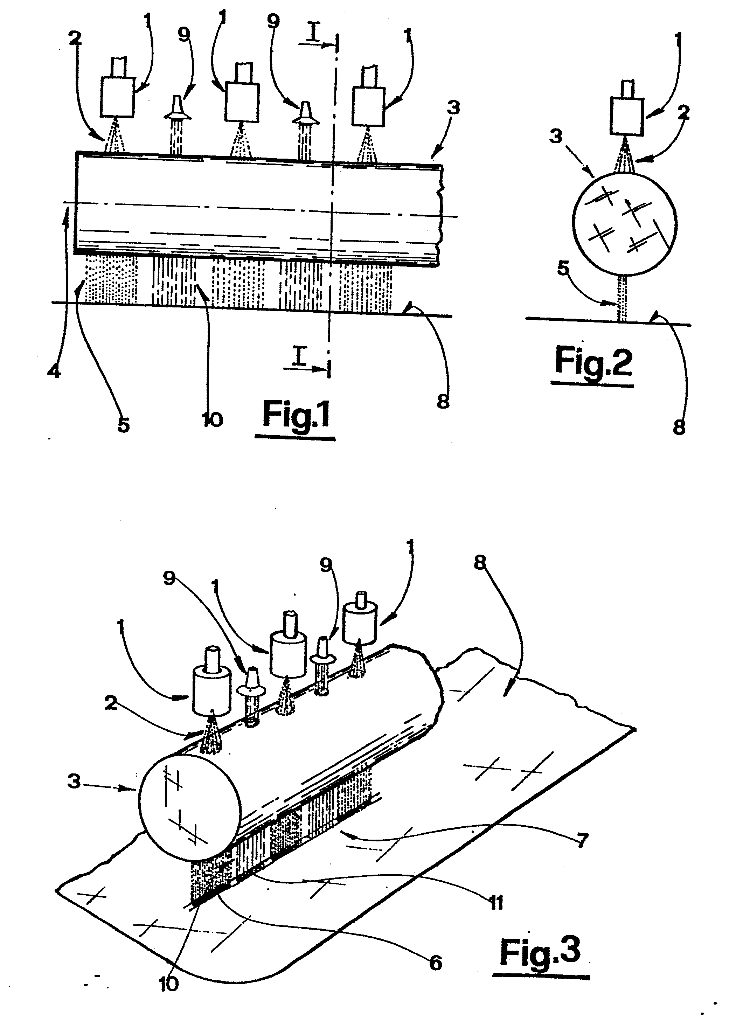

Fig 3 illustrates the cross-sections 6 of the emergent beams 5 and the section of the strip 7 produced. The angle of incidence of laser beams 5 focused thus by the cylindrical lens 3 onto a surface 8 beneath, is 90°.

The semiconductor laser devices 1 in question emit beams 2 in the infrared part of the spectrum (0.9μm or thereabouts), signifying that radiation will be invisible, and in order to enable the user to see the thin strip 7 on which the total laser radiation focused by the cylindrical lens 3 is incident, the invention envisages light sources 9 located, say, in alternation with the laser devices 1, that will emit radiation within the visible part of the spectrum. The light sources 9 are arranged in alignment with and at either side of the single semiconductor laser devices 1 in such a way that the visible radiation emitted from each source passes through the cylindrical lens 3 and is focused into a relative emergent beam 10 the typical cross-section 11 of which will exhibit one dimension (length) significantly greater than the other (width); such emergent beams 10 of light are aligned one with the next and thrown onto the strip 7 formed by the focused laser beams 5, the result being that the strip 7 is rendered visible on the surface 8 by the presence of the light beams 10, the cross-sections 11 of which will coincide with the strip and superimpose it, at least in part.

Both the semiconductor laser devices 1 and the light sources 9 are associated with the cylindrical lens 3 in immovable fashion, creating an assembly such as renders the use of the equipment easy and practical. More precisely, coverage of a given surface extending the length of the cylindrical lens 3 can be implemented simply by traversing the assembly at right angles to the axis of the lens 3, thereby dispensing with the two reciprocally perpendicular movements normally required for laser beams produced by prior art equipment, which are of substantially circular cross-section.

What is more, the adoption of a battery of semiconductor laser devices 1, which produce an output per unit of surface area higher than is obtainable from conventional laser equipment for medical use, brings the additional advantage, amongst others, of faster therapy.

The embodiment thus described is illustrated with a lens of cylindrical shape; alernatively, such a lens could be of elliptical cross-section, or faceted, or might exhibit any given shape such as will focus the incident laser beams as described in the foregoing, whilst remaining equivalent both in terms of the art and in the light of the protection afforded by appended claims.

Claims

1) Laser equipment for medical use, characterized:
in that it comprises a number of laser devices (1)
ranged in succession one alongside the next in such
a way that the relative beams (2) emitted are disposed with their axes substantially parallel and are
directed at a focusing medium of elongated form the
longitudinal axis of which lies substantially perpendicular to the axes of the beams; and,
in that the purpose of such a medium is to focus the
incident laser beams (2) in such a way as to produce
a corresponding number of emergent beams (5) the
respective cross-sections (6) of which each exhibit
one dimension significantly greater than the other;
wherein such cross-sections are located in close
proximity one to the next and aligned in succession
through the significantly greater dimension so as to
form what is substantially one continuous section
having the appearance of a thin strip (7).

2) Laser equipment as in claim 1, wherein the focusing
medium is embodied as a cylindrical lens (3), and
the laser devices (1) are disposed one alongside the
next in such a way that the axes of the relative
beams (2) emitted lie within the median plane containing the longitudinal axis of such a cylindrical
lens.

3)   Laser equipment as in claim 2, wherein the laser devices (1) are of the semiconductor type.

4)   Laser equipment as in claim 3, characterized in that it comprises a number of light sources (9), located in alternation with the laser devices, wherein each such source emits radiation within the visible part of the spectrum which passes through the cylindrical lens (3) and is focused into an emergent beam (10) the typical cross-section (11) of which exhibits one dimension significantly greater than the other, and wherein the cross-sections of such visible beams are aligned one with the next and thrown onto the strip (7) formed by the focused laser beams (5), thereby superimposing it at least in part.

**Fig.1**

**Fig.2**

**Fig.3**